(19) Europäisches Patentamt
European Patent Office
Office européen des brevets

(11) Veröffentlichungsnummer: **0 271 750**
**B1**

# (12) EUROPÄISCHE PATENTSCHRIFT

(45) Veröffentlichungstag der Patentschrift: **31.10.90**

(51) Int. Cl.[5]: **C07D 265/30**, C07D 295/02, A01N 43/84, A01N 43/34

(21) Anmeldenummer: **87117468.6**

(22) Anmeldetag: **26.11.87**

## (54) Fungizide Cyclohexylamine.

(30) Priorität: **17.12.86 DE 3643009**

(43) Veröffentlichungstag der Anmeldung:
**22.06.88 Patentblatt 88/25**

(45) Bekanntmachung des Hinweises auf die Patenterteilung:
**31.10.90 Patentblatt 90/44**

(84) Benannte Vertragsstaaten:
**AT BE CH DE ES FR GB GR IT LI NL SE**

(56) Entgegenhaltungen:
**EP-A- 0 029 617**
**DE-A- 3 321 712**
**DE-B- 1 214 471**

**CHEMICAL ABSTRACTS, Band 63, Nr. 1, 5. Juli 1965, Columbus, Ohio, USA; K.H. KOENIG et al.: "Novel fungicides" Spalte 1784, Zusammenfassung-Nr. 1 784b**

(73) Patentinhaber: **BASF Aktiengesellschaft, Carl-Bosch-Strasse 38, D-6700 Ludwigshafen(DE)**

(72) Erfinder: **Buschmann, Ernst, Dr., Georg-Ludwig-Krebs-Strasse 10, D-6700 Ludwigshafen(DE)**
Erfinder: **Goetz, Norbert, Dr., Schoefferstrasse 25, D-6520 Worms 1(DE)**
Erfinder: **Zipperer, Bernhard, Dr., Am Herrgottsacker 6, D-6716 Dirmstein(DE)**
Erfinder: **Ammermann, Eberhard, Dr., Sachsenstrasse 3, D-6700 Ludwigshafen(DE)**
Erfinder: **Pommer, Ernst-Heinrich, Dr., Berliner Platz 7, D-6703 Limburgerhof(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft tertiäre, 4-substituierte Cyclohexylamine, sowie Verfahren zu ihrer Herstellung, ihre Verwendung als Fungizide, fungizide Mischungen sowie Verfahren zur Bekämpfung von Schadpilzen mit diesen Wirkstoffen.

Es ist bekannt, N-Cyclododecyl-2, 6-trans-dimethylmorpholin als Fungizid zu verwenden (DE 3 321 712). Es ist ferner bekannt, N-(4'-Cyclohexyl-cyclohexyl)-2,6-dimethylmorpholin als Fungizid zu verwenden (C.A.Nr. 1541-88-4). Es sind auch alkylsubstituierte N-Cyclohexyl-2,6-dimethylmorpholine mit fungizider Wirkung bekannt (DE 1 214 471). Ihre fungizide Wirkung, insbesondere die Breite des Wirkungsspektrums, ist jedoch nicht befriedigend.

Es wurde nun gefunden, daß in 4-Stellung substituierte Cyclohexylamine der Formel I,

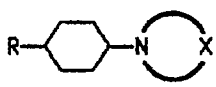

I,

in der R die Gruppe $CR^1R^2R^3$ mit insgesamt 6 bis 20 C-Atom bedeutet, in welcher $R^1$, $R^2$ und $R^3$ gleich oder verschieden sind und Wasserstoff verzweigtes oder unverzweigtes, gegebenenfalls durch Hydroxy oder $C_6$-Cycloalkyl substituiertes $C_1$-$C_8$-Alkyl, $C_1$-$C_8$-Alkoxy, $C_1$-$C_8$-Alkylthio oder $C_3$-$C_6$-Cycloalkyl bedeuten, mit der Maßgabe, daß höchstens einer der Substituenten $R^1$, $R^2$ oder $R^3$ Wasserstoff sein darf,

X eine Alkylengruppe mit vier bis sechs C-Atomen bedeutet, die gemeinsam mit dem N-Atom einen fünf- bis siebengliedrigen heterocyclischen Ring bildet, in dem gegebenenfalls bis zu zwei C-Atome durch O-, N- oder S-Atome ersetzt sein können und der durch ein bis drei geradkettige oder verzweigte Alkyl- oder Arylalkylreste mit jeweils 1 bis 10 C-Atomen substituiert sein kann und ihre Salze bei guter Pflanzenverträglichkeit ein breites fungizides Wirkungsspektrum aufweisen.

Unter Salzen sind die pflanzenveträglichen Salze mit beliebigen anorganischen oder organischen Säuren zu verstehen, z.B. mit Chlorwasserstoff, Bromwasserstoff, Schwefelsäure, Salpetersäure, Essigsäure, höheren Fettsäuren, z.B. Palmitinsäure, Arylsulfonsäuren, z.B. Dodecylbenzolsulfonsäure.

Die Gruppe $CR^1R^2R^3$ bedeutet beispielsweise:
n-Hex-2-yl, n-Hex-3-yl, 2-Methylpent-2-yl, 2,4,4-Trimethyl-pent-2-yl, 2-Methyl-hex-2-yl, 2-Methyhept-2-yl, 2,4-Dimethyl-oent-2-yl, 1-Cyclohexylethyl, 2-Cyclohexyl-prop-2-yl, 1-Cyclohexyl-prop-2-yl, 4-Cyclohexylbut-2-yl, 4-Cyclohexyl-2-methyl-but-2-yl, 2-Ethoxybut-2-yl.

Die Gruppe X bedeutet beispielsweise zusammen mit dem Stickstoffatom, dessen Substituent sie ist, den Rest von Pyrrolidin, Mono-, Di- oder Trimethylpyrrolidin, Piperidin, Mono-, Di-, Tri- oder Tetramethylpiperidin, Ethylpiperidin, Propylpiperidin, tert.-Butylpiperidin, Benzylpiperidin, Morpholin, Mono-, Di- oder Trimethylmorpholin, Thiomorpholin, Dimethylthiomorpholin, Piperazin, Ethylpiperazin, Propylpiperazin, tert.-Butylpiperazin, Hexamethylenimin.

Die neuen Amine der Formel 1 enthalten ggf. chirale Zentren. Sie werden im allgemeinen als Racemate und gegebenenfalls als Diastereomerengemische erhalten. Einheitliche Diastereomere lassen sich beispielsweise durch Destillation, Säulenchromatographie oder aufgrund von Löslichkeitsunterschieden in reiner Form isolieren. Aus solchen gereinigten Diastereomeren kann man mit bekannten Methoden einheitliche Racemate und Enantiomere erhalten. Alle diese Verbindungen und Gemische werden von der vorliegenden Erfindung umfaßt. Für die Anwendung der neuen Amine als Fungizide sind sowohl die einheitlichen Diastereomeren bzw. Enatiomeren wie auch deren bei der Synthese anfallenden Gemische geeignet. Bevorzugt werden die letzteren verwendet.

Man kann die neuen Amine z. B. dadurch erhalten, daß man die entsprechenden primären Amine der Formel II mit bifunktionellen Alkylierungsmitteln umsetzt.

Als Alkylierungsmittel kommen z. B. in Frage:
1,4-Dichlor(Dibrom)butan, 1,5-Dichlor(Dibrom)pentan, 1,6-Dichlor(Dibrom)hexan, Di-(2-chlorethyl)ether Di(2-chlorethyl)sulfid, Di-(2-chlorethyl)amin, Di-(2-chlorethyl)methyl- bzw. -ethyl-, -propyl-, -tert.-buylamin, Di-(1-chlor-2-propyl)ether, Di-(1-chlor-2-propyl)sulfid, Di-(1-chlor-2-propyl)methyl-, bzw. -ethyl-, -propyl-, -tert.-butyl-amin.

Die Alkylierungen werden ggf. in einem Verdünnungsmittel und in Gegenwart einer Hilfsbase durchgeführt.

Als Verdünnungsmittel eignen sich beispielsweise polare Lösungsmittel wie Methanol, Ethanol, Propanole, Butanole, Cyclohexanol, Aceton, Methylethylketon, Acetonitril, Dimethylformamid, N-Methylpyrrolidon, Ethylacetat, Nitromethan, Dimethylsulfoxid, Dioxan, Tetrahydrofuran.

Als Hilfsbasen für die Umsetzung zu Aminen der Formel I können alle üblichen Säurebindemittel verwendet werden. Hierzu gehören vorzugsweise Alkali-oder Erdalkalioxide, -hydroxide oder -carbonate, tertiäre Amine und basische Ionenaustauscher.

Beispielsweise kommen als basische Verbindungen in Frage:
Kaliumhydroxid, Natriumhydroxid, Calciumhydroxid, Bariumhydroxid, Calciumoxid, Bariumoxid, Magnesiumoxid, Kaliumcarbonat, Natriumcarbonat, Triethylamin, Tri-n-propylamin, Tri-n-butylamin, Ethyl-diisopropylamin, N,N-Dimethylcyclohexylamin, Puridin, Methylpyridine, Chinolin, Isochinolin, 2,6-Lutidin, 2,4,6-Kollidin.

Eine andere Möglichkeit zur Herstellung der Amine besteht darin, daß man die entsprechenden primären Amine II gemäß Anspruch 2 ggf. schrittweise mit Oxiranen alkyliert und die entstehenden Bis-(β-hydroxyalkyl)amine zu den entsprechenden Morpholinen cyclisiert.

Als Oxirane kommen hierbei vorzugsweise Ethylenoxid, Propylenoxid und Isobutylenoxid zum Einsatz. Die Alkylierung wird zweckmäßig in Gegenwart katalytischer oder stöchiometrischer Mengen einer Protonenquelle, z. B. Wasser, Ethanol, 1- oder 2-Propanol, n- oder tert.-Butanol, bei Temperaturen zwischen 0 und 150°C, vorzugsweise zwischen 20 und 100°C, drucklos oder ggf. unter Druck durchgeführt.

Die Cyclisierung der Bis-(β-hydroxyalkyl)amine zu den erfindungsgemäßen Morpholinen erfolgt zweckmäßig in Gegenwart einer starken Säure, z. B. Schwefelsäure, vorzugsweise bei Temperaturen von +50 bis +150°C.

Eine andere Möglichkeit zur Herstellung der Amine besteht darin, daß man die entsprechenden Cyclohexanone III gemäß Anspruch 2 mit sekundären Aminen der Formel IV zu Enaminen umsetzt und diese zu den Amine I hydriert, oder die entsprechenden Cyclohexanone III mit den sekundären Aminen IV gemäß Anspruch 2 in Gegenwart eines Reduktionsmittels direkt in die Amine I überführt.

Die Cyclohexanone III sind bekannte Verbindungen und nach bekannten Verfahren, z.B. aus den entsprechenden Phenolen, herstellbar.

Die cyclischen, sekundären Amine IV sind bekannte Verbindungen und vielfach im Handel erhältlich.

Die Herstellungsbedingungen von Enaminen aus den Cyclohexanonen III und den sekundären Aminen IV sind in der Literatur beschrieben (vgl. z.B. J. Smuskovicz: Enamines, Adv. Org. Chem. 4, 1 (1963); A.G. Cook: Enamines, M. Dekker, New York 1969; Houben-Weyl, Methoden der Org. Chemie, Bd. XI/1 (1957), S. 170 f).

Gleiches gilt auch fur die Hydrierung der Enamine zu den entsprechenden Aminen.

Auch die direkte reduktive Aminierung der Cyclohexanone III mit den sekundären Aminen IV zu den erfindungsgemäßen Aminen I erfolgt nach an sich bekannten Verfahren.

Als Reduktionsmittel kommen hierbei vor allem Wasserstoff (Houben-Weyl, Methoden der Organischen Chemie, Bd. XI/1 (1957), S. 602 f.), Ameisensaure (ibid., S. 648 f) und komplexe Hydride, vorzugsweise Natriumcyanborhydrid (Synthesis 1975, 135; Org. Prep. Procd. Int. 11 (1979) 201) in Frage.

Beispiel 1

2,6-Dimethyl-4-[4-(2-cyclohexyl-prop-2-yl)cyclohexyl]-morpholin

Eine Lösung von 23,3 g (0,1 mol) 4-(2-Cyclohexylprop-2-yl)cyclohexylamin und 17,1 g (0,1 mol) Di-chlor-2-propyl)ether in 200 ml Cyclohexanol wird mit 41,4 g (0,3 mol) Kaliumcarbonat versetzt und unter Rühren 8 Stunden am Rückfluß erhitzt. Nach Abkühlen rührt man ca. 100 ml Wasser zu, trennt die organ. Phase ab und trocknet über wenig $Na_2O_4$. Der nach Abdestillieren des Cyclohexanols verbleibende Rückstand wird i. Vak. fraktioniert destilliert. Mit Sdp. 160-164°C/0, 3 mba (0,2 mm) erhält man 16 g (50% d.Th.) der Titelverbindung als farbloses Öl (Isomerengemisch; Verbindung Nr. 1).

Beispiel 2

2,6-Dimethyl-4-[4-(2,4,4-trimethyl-pent-2yl)cyclohex-1-yl]-morpholin

Zu 211 g (1,0 mol)4-(2,4;4-trimethyl-pent-2-yl)cyclohexylamin (cis-trans-Gemisch) und 6 g (0,33 mol) Wasser tropft man bei 60-70°C unter Rühren 240 g (4,0 mol) Propylenoxid. Man erwärmt auf 90-95°C und gibt insgesamt 7 mal alle 6 Stdn. jeweils 116 g (2,0 mol) Propylenoxid zu. Die Umsetzung kann gaschromatographisch kontrolliert werden.

Ohne weitere Aufarbeitung wird i. Vak. über eine Fraktionskolonne destilliert. Bei 190-195°C/0,8 mba 0,6 mm gehen ca. 200 g eines 9:1-Gemisches aus Bis-(2-Hydroxyopropyl)-4-(2,4,4-trimethyl-2-pentyl cyclohexylamin und dem monoalkylierten Amin über. Durch erneute Destillation erhält man daraus 171 g reines tertiäres Amin.

Dieses wird mit der gleichen Gewichtsmenge konz. Schwefelsäure versetzt und 4 Std. bei 100-110°C gerührt. Unter Kühlung wird mit verd. NaOH auf pH 12 gebracht und das Morpholin mehrfach mit Dichlormethan extrahiert. Die getrocknete organ. Phase wird eingeengt und i. Vak. destilliert. Man erhält 120g der Titelverbindung als farbloses Öl vom Sdp. 165-168°C/3 mbar (2mm) (ca. 39% d.Th.; Verbindung Nr. 2).

In entsprechender Weise werden die folgenden Verbindungen erhalten:

Tabelle

| Beispiel Nr. | $R^1$ | $R^2$ | $R^3$ | X | Phys. Daten (Kp/Fp) |
|---|---|---|---|---|---|
| 1 | $CH_3$ | $CH_3$ | cyclo-$C_6H_{11}$ | $-CH_2CH(CH_3)OCH(CH_3)CH_2$ | 160-164°C/0,3 mbar (0,2mm) |
| 2 | $CH_3$ | $CH_3$ | $(CH_3)_3CCH_2$ | $-CH_2CH(CH_3)OCH(CH_3)CH_2-$ | 165-168°C/2,7 mbar (2,0mm) |
| 3 | $CH_3$ | $CH_3$ | cyclo-$C_6H_{11}$ | $-CH_2CH_2OCH_2CH_2-$ | |
| 4 | $CH_3$ | $CH_3$ | cyclo-$C_6H_{11}$ | $-(CH_2)_6-$ | |
| 5 | $CH_3$ | $CH_3$ | cyclo-$C_6H_{11}$ | $-(CH_2)_5-$ | |
| 6 | $CH_3$ | $CH_3$ | cyclo-$C_6H_{11}$ | $-(CH_2)_4$ | |
| 7 | $CH_3$ | $CH_3$ | $(CH_3)_3CCH_2$ | $-CH_2CH_2OCH_2CH_2-$ | |
| 8 | $CH_3$ | $CH_3$ | $(CH_3)_3CCH_2$ | $-(CH_2)_6-$ | |
| 9 | $CH_3$ | $CH_3$ | $(CH_3)_3CCH_2$ | $-(CH_2)_5-$ | |
| 10 | $CH_3$ | $CH_3$ | $(CH_3)_3CCH_2$ | $-(CH_2)_4-$ | |
| 11 | $CH_3$ | $CH_3$ | $CH_3CH(CH_3)CH_2$ | $-(CH_2)_4-$ | |
| 12 | $CH_3$ | $CH_3$ | $CH_3CH(CH_3)CH_2$ | $-(CH_2)_5-$ | |
| 13 | $CH_3$ | $CH_3$ | $CH_3CH(CH_3)CH_2$ | $-(CH_2)_6-$ | |
| 14 | $CH_3$ | $CH_3$ | $CH_3CH(CH_3)CH_2$ | $-CH_2CH_2OCH_2CH_2-$ | |
| 15 | $CH_3$ | $CH_3$ | $CH_3CH(CH_3)CH_2$ | $-CH_2CH(CH_3)OCH(CH_3)CH_2-$ | 148-154°C/0,5 mbar (0,4mm) |
| 16 | $CH_3$ | $CH_3$ | n-$C_5H_{11}$ | $-CH_2CH(CH_3)OCH(CH_3)CH_2-$ | 156-160°C/0,9 mbar (0,7mm) |
| 17 | $CH_3$ | $CH_3$ | n-$C_5H_{11}$ | $-CH_2CH_2OCH_2CH_2-$ | |
| 18 | $CH_3$ | $CH_3$ | n-$C_5H_{11}$ | $-(CH_2)_6-$ | |
| 19 | $CH_3$ | $CH_3$ | n-$C_5H_{11}$ | $-(CH_2)_5-$ | |
| 20 | $CH_3$ | $CH_3$ | n-$C_5H_{11}$ | $-(CH_2)_4-$ | |
| 21 | cyclo-$C_6H_{11}CH_2CH_2$ | $CH_3$ | H | $-(CH_2)_4-$ | |
| 22 | cyclo-$C_6H_{11}CH_2CH_2$ | $CH_3$ | H | $-(CH_2)_5-$ | |
| 23 | cyclo-$C_6H_{11}CH_2CH_2$ | $CH_3$ | H | $-(CH_2)_6-$ | |
| 24 | cyclo-$C_6H_{11}CH_2CH_2$ | $CH_3$ | H | $-CH_2CH_2OCH_2CH_2-$ | |
| 25 | cyclo-$C_6H_{11}CH_2CH_2$ | $CH_3$ | H | $-CH_2CH(CH_3)OCH(CH_3)CH_2-$ | 170°C /0,4 mbar (0,3mm) |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle,
Ustilago-Arten an Getreide und Zuckerrohr,
Venturia inaequalis (Schorf) an Äpfeln,
Pyrenophora teres an Gerste
Botrytis cinerea (Grauschimmel) an Erdbeeren, Reben,
Cercospora arachidicola an Erdnüssen,
Mycosphaerella-Arten in Bananen,
Pseudocercosporella herpotrichoides an Weizen, Gerste,
Pyricularia oryzae an Reis,
Phytophthora infestans an Kartoffeln und Tomaten,
Plasmopara viticola an Reben sowie
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, chlorierte Aromaten (z.B. Chlorbenzole), Paraffine (z.B. Erdöfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden zum Beispiel gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet. beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III.20 Gew.-Teile der Verbindung Nr. 15 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teilen der Verbindung Nr. 25 werden in einer Mischung gelöst, die aus 25 Gew.-Teilen Cyclohexanol, 65 Gew.-Teilen einer Mineralölfraktion vom Siedepunkt 210 bis 280°C and 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teilen der Verbindung Nr. 16 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigen Kieselsäuregel gut vermischt und in einer Hammermühle vermahlen. Durch feines Verteilen der Mischung in Wasser erhält man eine Spritzbrühe.

VI. 3 Gew.-Teilen der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII.30 Gew.-Teilen der Verbindung Nr. 15 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teilen der Verbindung Nr. 2 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Wirkungsbeispiele

Als Vergleichswirkstoff (A) wurde N-Cyclododecyl-2,6-trans-dimethylmorpholin und als Vergleichswirkstoff (B) N-(4′-Cyclohexyl-cyclohexyl)2,6-dimethylmorpholin benutzt.

Anwendungsbeispiel 1

Wirksamkeit gegen Pyrenophora teres

Gerstenkeimlinge der Sorte "Asse" wurden im Zweiblattstadium mit wäßrigen Suspensionen, die 80% (Gew.%) Wirkstoff und 20% Emulgator in der Trockensubstanz enthielten, tropfnaß gespritzt. Nach 24 Stunden wurden die Pflanzen mit einer Sporensuspension des Pilzes Pyrenopohra teres inoculiert und für 48 Stunden in eine Klimakammer mit hoher Luftfeuchtigkeit bei 18°C gestellt. Anschließend wurden die Pflanzen im Gewächshaus bei 20 bis 22°C und 70% relativer Luftfeuchtigkeit für weitere 5 Tage kultiviert. Dann wurde das Ausmaß des Pilzbefalls ermittelt.

Das Ergebnis des Versuches zeigt, daß nach der Anwendung einer 0,05%igen (Gew.%) Spritzbrühe beispielsweise die Verbindungen Nr. 1, 2, 15 und 25 eine sehr gute fungizide Wirkung zeigten (90%), während der bekannte Wirkstoff B unwirksam war (0%).

Anwendungsbeispiel 2

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Bei diesem Versuch war die fungizide Wirkung nach Behandlung mit 0,025%iger Wirkstoffzubereitung beispielsweise bei den Verbindungen 1, 2, 15 und 25 sehr gut (90 %), während sie bei den Vergleichswirkstoffen A und B nur mäßig war (60 %).

Anwendungsbeispiel 3

Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielt, besprüht und 24 Stunden nach dem Antrocknen des Spritzbelages mit Oidien (Sporen) des Weizenmehltaus (Erysiphe graminis f.sp. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Bei diesem Versuch wurde mit 0,0125%igen Wirkstoffzubereitungen beispielsweise der Verbindungen 1, 2, 15 und 16 eine sehr gute fungizide Wirkung erzielt (90 %), während die Vergleichswirkstoffe A und B eine mäßige fungizide Wirkung zeigten (60 %).

## Patentansprüche

1. 4-substituierte Cyclohexylamine der Formel I,

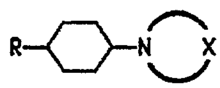

I,

in der R die Gruppe $CR^1R^2R^3$ mit insgesamt 6 - 20 C-Atomen bedeutet, in welcher $R^1$, $R^2$ und $R^3$ gleich oder verschieden sind und Wasserstoff, verzweigtes oder unverzweigtes, ggf. durch Hydroxy oder $C_6$-Cycloalkyl substituiertes $C_1$-$C_8$-Alkyl, $C_1$-$C_8$-Alkoxy, $C_1$-$C_8$-Alkylthio oder $C_3$-$C_6$-Cycloalkyl bedeuten, mit der Maßgabe, daß höchstens einer der Substituenten $R^1$, $R^2$ oder $R^3$ Wasserstoff sein darf, in der X eine Alkylengruppe mit vier bis sechs C-Atomen bedeutet, die gemeinsam mit dem N-Atom einen fünf- bis siebengliedrigen heterocyclischen Ring bildet, in dem bis zu 2 C- Atome durch O-, N- oder S-Atome ersetzt sein können und der durch 1 bis 3 geradkettige oder verzweigte Alkyl- oder Arylalkylreste mit jeweils 1 bis 10 C-Atomen substituiert sein kann, sowie deren Salze.

2. Verfahren zur Herstellung der Cyclohexylamine der Formel I gemäß Anspruch 1, dadurch gekennzeichnet, daß man
A) ein Cyclohexylamin der Formel II

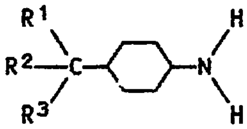

II

a) mit einem bifunktionellen Alkylierungsmittel alkyliert oder gegebenenfalls
b) mit monofunktionellen Alkylierungsmitteln zweifach alkyliert und aus den Alkylketten anschließend einen Ring gemäß Formel I aufbaut,
oder
B) ein Cyclohexanon der Formel III

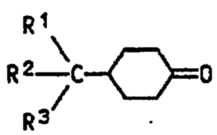

III

mit einem sekundären Amin der Formel IV

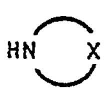

IV.

in denen $R^1$, $R^2$, $R^3$ und X die in Anspruch 1 genannte Bedeutung haben, umsetzt und nachfolgend hydriert oder in Gegenwart eines Reduktionsmittels reduktiv aminiert.

Fungizides Mittel, enthaltend ein 4-substituiertes Cyclohexylamin der Formel I

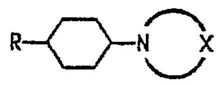

I,

in der R die Gruppe $CR^1R^2R^3$ mit insgesamt 6–20 C-Atomen bedeutet, in welcher $R^1$, $R^2$ und $R^3$ gleich oder verschieden sind und Wasserstoff, verzweigtes oder unverzweigtes, ggf. durch Hydroxy oder $C_6$-Cycloalkyl substituiertes $C_1$-$C_8$-Alkyl, $C_1$-$C_8$-Alkoxy, $C_1$-$C_8$-Alkylthio oder $C_3$-$C_6$-Cycloalkyl bedeuten, mit der Maßgabe, daß höchstens einer der Substituenten $R^1$, $R^2$ oder $R^3$ Wasserstoff sein darf,

in der X eine Alkylengruppe mit vier bis sechs C-Atomen bedeutet, die gemeinsam mit dem N-Atom einen fünf- bis siebengliedrigen heterocyclischen Ring bildet, in dem bis zu 2 C-Atome durch 0-, N- oder S-Atome ersetzt sein können und der durch 1 bis 3 geradkettige oder verzweigte Alkyl- oder Arylalkylreste mit jeweils 1 bis 10 C-Atomen substituiert sein kann,

oder dessen Salz und einen festen oder flüssigen Trägerstoff.

4. Verfahren zur Herstellung von Fungiziden, dadurch gekennzeichnet, daß man eine Verbindung der Formel I gemäß Anspruch 1 mit einem festen oder flüssigen Trägerstoff mischt.

5. Verfahren zur Bekämpfung von Pilzen, dadurch gekennzeichnet, daß man ein 4-substituiertes Cyclohexylamin der Formel I

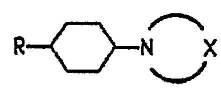

I,

in der R die Gruppe $CR^1R^2R^3$ mit insgesamt 6–20 C-Atomen bedeutet, in welcher $R^1$, $R^2$ und $R^3$ gleich oder verschieden sind und Wasserstoff, verzweigtes oder unverzweigtes, ggf. durch Hydroxy oder $C_6$-Cycloalkyl substituiertes $C_1$-$C_8$-Alkyl, $C_1$-$C_8$-Alkoxy, $C_1$-$C_8$-Alkylthio oder $C_3$-$C_6$-Cycloalkyl bedeuten, mit der Maßgabe, daß höchstens einer der Substituenten $R^1$, $R^2$ oder $R^3$ Wasserstoff sein darf,
in der X eine Alkylengruppe mit vier bis sechs C-Atomen bedeutet, die gemeinsam mit dem N-Atom einen fünf- bis siebengliedrigen heterocyclischen Ring bildet, in dem bis zu 2 C-Atome durch O-, N- oder S-Atome ersetzt sein können und der durch 1 bis 3 geradkettige oder verzweigte Alkyl- oder Arylalkylreste mit jeweils 1 bis 10 C-Atomen substituiert sein kann,
oder deren Salz auf Pilze oder auf durch Pilzbefall bedrohte Materialien, Flächen, Pflanzen oder Saatgut einwirken läßt.

6. Verbindung gemäß Anspruch 1, dadurch gekennzeichnet, daß $R^1$ und $R^2$ Methyl, $R^3$ den Rest $-CH_2C(CH_3)_3$ oder Cyclohexyl und X den Rest $-CH_2-CH(CH_3)_3-O-CH(CH_3)-CH_2-$ bedeuten.

7. Verbindung gemäß Anspruch 1, dadurch gekennzeichnet, daß $R^1$ und $R^2$ Methyl, $R^3$ den Rest $-CH_2-CH(CH_3)_2$ oder n-Pentyl und X den Rest $-CH_2-CH(CH_3)-O-CH(CH_3)CH_2$ bedeuten.

## Claims

1. 4-Substituted cyclohexylamines of the formula I

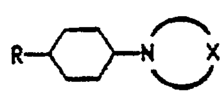

I

where R is the $CR^1R^2R^3$ group having a total of 6-20 carbon atoms, where $R^1$, $R^2$ and $R^3$ are identical or different and are hydrogen, branched or aunbranched, unsubstituted or hydroxyl- or $C_6$-cycloalkyl-substituted $C_1$-$C_8$-alkyl, $C_1$-$C_8$-alkoxy, $C_1$-$C_8$-alkylthio or $C_3$-$C_6$-cycloalkyl, with the proviso that at most one of the substitutents $R^1$, $R^2$ and $R^3$ may be hydrogen,

and where X is an alkylene group having from four to six carbon atoms which, together with the nitrogen atom, forms a 5- to 7- membered heterocyclic ring in which up to 2 carbon atoms may be replaced by oxygen, nitrogen or sulfur atoms and which may be substituted by from 1 to 3 straight-chain or branched alkyl or arylalkyl radicals, in each case having from 1 to 10 carbon atoms, and the salts thereof.

2. A process for the preparation of the cyclohexylamines of the formula I as claimed in claim 1, wherein
A) a cyclohexylamine of the formula II

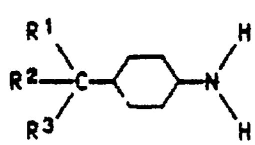

II

a) is alkylated using a bifunctional alkylating agent, or, if desired,

b) is dialkylated using a monofunctional alkylating agent, and a ring as in the formula I is subsequently built up from the alkyl chains,

or

B) a cyclohexanone of the formula III

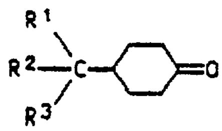

III

is reacted with a secondary amine of the formula IV

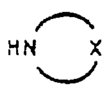

IV

where $R^1$, $R^2$, $R^3$ and X are as defined in claim 1, and the product is subsequently hydrogenated or subjected to reductive amination in the presence of a reducing agent.

3. A fingicide containing a 4-substituted cyclohexylamine of the formula I

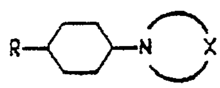

I

Where R is the $CR^1R^2R^3$ group having a total of 6-20 carbon atoms, where $R^1$, $R^2$ and $R^3$ are identical or different and arer hydrogen, branched or unbranched, unsubstituted or hydroxyl- or $C_6$-cycloalkyl-substituted $C_1$-$C_8$-alkyl, $C_1$-$C_8$-alkoxyl, $C_1$-$C_8$-alkylthio or $C_3$-$C_6$-cycloalkyl, with the proviso that at most one of the substitutents $R^1$, $R^2$ and $R^3$ may be hydrogen,

and where X is an alkylene group having from four to six carbon atoms which, together with the nitrogen atom, forms a five- to seven-membered heterocyclic ring in which up to 2 carbon atoms may be replaced by oxygen, nitrogen or sulfur atoms and which may be substituted by from 1 to 3 straight-chain or branched alkyl or arylalkyl radicals, in each case having from 1 to 10 carbon atoms, or a salt thereof, and a solid or liquid carrier.

4. A process for the preperation of fungicides, wherein a compound of the formula I as claimed in claim 1 is mixed with a solid or liquid carrier.

5. A process for controlling fungi, wherein a 4-substituted cyclohexylamine of the formula I

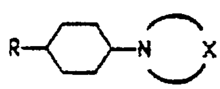

I

where R is the $CR^1R^2R^3$ group having a total of 6-20 carbon atoms, where $R^1$, $R^2$ and $R^3$ are identical or different and are hydrogen, branched or unbranched, unsubstituted or hydroxyl- or $C_6$-cycloalkyl- substituted $C_1$-$C_8$-alkyl, $C_1$-$C_8$-alkoxy, $C_1$-$C_8$-alkylthio, with the proviso that at most one of the substitutents $R^1$, $R^2$ and $R^3$ may be hydrogen,

and where X is an alkylene group having from four to six carbon atoms which, together with the nitrogen atom, forms a five- to seven-membered heterocyclic ring in which up to 2 carbon atoms may be replaced by oxygen, nitrogen or sulfur atoms and which may be substituted by from 1 to 3 straight-chain or branched alkyl or arylakyl radicals, in each case having from 1 to 10 carbon atoms, or a salt thereof, is allowed to act on fugi or materials, areas, plants or seeds threatened by fungal infestation.

6. A compound as claimed in claim1, wherein $R^1$ and $R^2$ are methyl, $R^3$ is the $CH_2C(CH_3)_3$ radical or cyclohexyl, and X is the $-CH_2-CH(CH_3)-O-CH(CH_3)-CH_2-$ radical.

7. A compound as claimed in claim 1, wherein $R^1$ and $R^2$ are methyl, $R^3$ is the $CH_2CH(CH_3)_2$ radical or n-pentyl, and X is the $-CH_2-CH(CH_3)-O-CH(CH_3)-CH_2-$ radical.

Revendications

1. Cyclohéxylamine substituée en 4, de la formule I

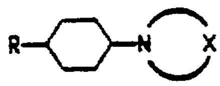

I,

dans laquelle R représente le groupe $CR^1R^2R^3$ ayant au total 6–20 atomes C, dans lequel $R^1$, $R^2$ et $R^3$ sont identiques ou différents et représentent hydrogène, alkyle en $C_1$–$C_8$, alcoxy en $C_1$–$C_8$, alkylthio en $C_1$–$C_8$ ou cycloalkyle en C3C6, ramifié ou non ramifié, éventuellement substitué par hydroxy ou cycloalkyle en $C_6$, sous réserve qu'au maximum un des substituants $R^1$, $R^2$ ou $R^3$ puisse être l'hydrogène, dans laquelle X représente un groupe alkylène ayant 4 à 6 atomes C, qui forme avec l'atome N un noyau hétérocyclique de 5 à 7 membres, dans lequel jusqu'à 2 atomes C peuvent être remplacés par des atomes 0, N ou Set qui peut être substitué par 1 à 3 restes alkyle ou arylalkyle à chaîne droite ou ramifiée, ayant chacun 1 à 10 atomes C, ainsi que leurs sels.

2. Procédé de préparation de la cyclohéxylamine de la formule I selon la revendication 1, caractérisé par le fait que

A) on alkyle une cyclohéxylamine de la formule II

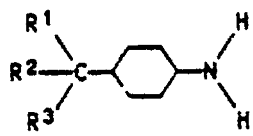

II

a) avec un agent d'alkylation bifonctionnel ou éventuellement

b) deux fois avec des agents d'alkylation monofonctionnel et des chaînes alkyle on forme ensuite un noyau selon la formule I ou

B) on fait réagir une cyclohexanone de la formule III

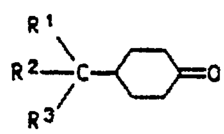

III

avec une amine secondaire de la formule IV

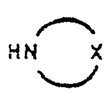

IV

dans laquelle $R^1$, $R^2$, $R^3$ et X ont les significations indiquées dans la revendication 1 et on hydrure ensuite ou on amine de façon réductive en présence d'un agent de réduction.

3. Agent fongicide, contenant une cyclohéxylamine substituée en 4, de la formule I

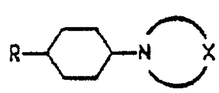

I

dans laquelle R représente le groupe $CR^1R^2R^3$ ayant au total 6–20 atomes C, dans lequel $R^1$, $R^2$ et $R^3$ sont identiques ou différents et représentent hydrogène, alkyle en $C_1$–$C_8$, alcoxy en $C_1$–$C_8$, alkylthio en $C_1$–$C_8$ ou cycloalkyle en $C_3$–$C_6$, ramifié ou non ramifié, éventuellement substitué par hydroxy oucycloalkyle en $C_6$, sous réserve qu'au maximum un des substituants $R^1$, $R^2$ ou $R^3$ puisse être l'hydrogène, dans laquelle X représente un groupe alkylène ayant 4 à 6 atomes C, qui forme avec l'atome N un noyau hétérocyclique de 5 à 7 membres, dans lequel jusqu'à 2 atomes C peuvent être remplacés par des atomes 0, N ou S et qui peut être substitué par 1 à 3 restes alkyle ou arylalkyle à chaîne droite ou ramifiée, ayant chacun 1 à 10 atomes C, ainsi que leurs sels et un support solide ou liquide.

4. Procédé de préparation de fongicides, caractérisé par le fait qu'on mélange un composé de formule I selon la revendication 1 avec un support solide ou liquide.

5. Procédé de lutte contre des champignons, caractérisé par le fait qu'on fait agir sur les champignons ou sur les matériaux, surfaces, plantes ou semences menacés par une attaque par champignons, une cyclohéxylamine substituée en 4, de la formule I

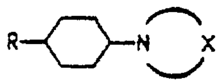

I

dans laquelle R représente le groupe $CR^1R^2R^3$ ayant au total 6–20 atomes C, dans lequel $R^1$, $R^2$ et $R^3$ sont identiques ou différents et représentent hydrogène, alkyle en $C_1$–$C_8$, alcoxy en $C_1$–$C_8$, alkylthio en $C_1$–$C_8$ ou cycloalkyle en $C_3$–$C_6$, ramifié ou non ramifié, éventuellement substitué par hydroxy ou cycloalkyle en $C_6$, sous réserve qu'au maximum un des substituants $R^1$, $R^2$ ou $R^3$ puisse être l'hydrogène, dans laquelle X représente un groupe alkylène ayant 4 à 6 atomes C, qui forme avec l'atome N un noyau hétérocyclique de 5 à 7 membres, dans lequel jusqu'à 2 atomes C peuvent être remplacés par des atomes O, N ou S et qui peut être substitué par 1 à 3 restes alkyle ou arylalkyle à chaîne droite ou ramifiée, ayant chacun 1 à 10 atomes C, ainsi que leurs sels.

6. Composé selon la revendication 1, caractérisé par le fait que $R^1$ et $R^2$ représentent méthyle, $R^3$, le reste $CH_2C(CH_3)_3$ ou cyclohéxyle et X, le reste $-CH_2-CH(CH_3)-O-CH(CH_3)-CH_2$.

7. Composé selon la revendication 1, caractérisé par le fait que $R^1$ et $R^2$ représentent méthyle, $R^3$, le reste $CH_2CH(CH_3)_2$ ou n-pentyle et X le reste $-CH_2CH(CH_3)-O-CH(CH_3)CH_2$.